# EUROPÄISCHE PATENTSCHRIFT

(11) **EP 2 925 384 B1**
(45) Veröffentlichungstag und Bekanntmachung des Hinweises auf die Patenterteilung: **05.09.2018**
(21) Anmeldenummer: 13798660.0
(22) Anmeldetag: 29.11.2013
(51) Int. Cl.: A61M 1/36, B01D 36/00, B01D 63/02, F16K 24/04

(54) **VORRICHTUNG ZUR RASCHEN ENTLÜFTUNG UND ENTLEERUNG EINES FILTERS**
DEVICE FOR QUICKLY VENTING AND DRAINING A FILTER
DISPOSITIF DE PURGE D'AIR ET DE VIDANGE RAPIDE D'UN FILTRE

(30) Priorität: 03.12.2012 DE 102012023504; 03.12.2012 US 201261732628 P
(43) Veröffentlichungstag der Anmeldung: 07.10.2015
(60) Teilanmeldung: 17184739.5
(73) Patentinhaber: Fresenius Medical Care Deutschland GmbH, 61352 Bad Homburg (DE)
(72) Erfinder: KONEGGER, Mario, A-6020 Innsbruck (AT)
(74) Vertreter: Dreyhsig, Jörg
(86) Internationale Anmeldenummer: PCT/EP2013/075103
(87) Internationale Veröffentlichungsnummer: WO 2014/086681

(56) Entgegenhaltungen:
- DE-C1- 4 027 531
- DE-U1- 8 603 781
- US-A- 4 104 004
- US-A- 4 981 154
- US-A1- 2011 108 482

## Beschreibung

Die Erfindung betrifft eine Vorrichtung zur Entlüftung eines Filters, bevorzugt eines Hohlfaserfilters, nach dem Oberbegriff des Anspruchs 1.

Filter werden in vielen weiten Bereichen der Industrie und in der Medizin zur Reinigung von Gasen und Flüssigkeiten eingesetzt. Die eingesetzten Filter sind dabei derart gestaltet, dass sie aus zwei Kammern bestehen, die voneinander mit einer semipermeablen Membran getrennt sind. Die semipermeable Membran ist dabei derart beschaffen, dass durch sie aus einer ersten Kammer Flüssigkeit in eine zweite Kammer gedrückt werden kann, während höhermolekulare Stoffe zurückgehalten werden.

Ein Anwendungsgebiet von Fluidfiltern sind Verfahren zur Fluidbehandlung oder Blutbehandlung, etwa der Hämodialyse oder der Peritonealdialyse.

Bei der Hämodialyse wird Blut in einem extrakorporalen Kreislauf kontinuierlich einem Patienten entnommen, durch einen Hämodialysator geleitet und dem Patienten wieder reinfundiert. Dabei wird ein Stoffaustausch durchgeführt, der dem der Nieren ähnlich ist. Der Hämodialysator besteht aus zwei durch eine semipermeable Membran getrennte Kammern, von denen die eine vom Blut und die andere von einer Reinigungsflüssigkeit - der Dialysierflüssigkeit - durchflossen wird. Die handelsüblichen Hämodialysatoren weisen hierfür meist Tausende von Hohlfasern auf, deren Wände die semipermeable Membran für die auszutauschenden Substanzen bilden. Das Blut wird durch den Innenraum der Hohlfasern geleitet, während die Dialysierflüssigkeit in meist gegenläufiger Richtung in den Hohlfaserzwischenraum eingespeist und abgeführt wird.

Die Dialysierflüssigkeit weist Konzentrationen von Blutinhaltsstoffen wie Elektrolyten auf, die in etwa denen eines gesunden Menschen entsprechen, damit die entsprechenden Konzentrationen im Blut auf einem normalen Niveau gehalten werden können. Aus dem Blut zu entfernende Stoffe wie zum Beispiel Kreatinin oder Harnstoff sind in der Dialysierflüssigkeit nicht enthalten, wodurch diese allein wegen des Konzentrationsgradienten an der Membran durch Diffusion aus dem Blut entfernt werden. Mit Hilfe eines Druckgradienten wird dem Blut überschüssiges Wasser durch Konvektion beziehungsweise Ultrafiltration entzogen. Der aus Konvektion und Diffusion kombinierte Entzug wird als Diafiltration bezeichnet.

Zur Steuerung derartiger Vorgänge werden Hämodialysegeräte eingesetzt, die zumeist auch die Zubereitung der Dialysierflüssigkeit aus Wasser und Konzentraten mit der richtigen Zusammensetzung und Temperatur sicherstellen. Bei der Hämodiafiltration wird dem Blut des Patienten während einer Hämodialysebehandlung über den Hämodialysator eine größere Menge Ultrafiltrat entzogen, die bis auf die insgesamt zu entziehende Flüssigkeitsmenge wieder durch Substitutionsflüssigkeit ersetzt wird. Bei modernen Geräten zur Behandlung des chronischen Nierenversagens wird hierfür die online aufbereitete Dialysierflüssigkeit verwendet, indem eine von dem Dialysierflüssigkeitskreislauf abzweigende Leitung mit einem oder mehreren Filterstufen versehen und mit dem extrakorporalen Blutkreislauf stromauf und/oder stromab des Hämodialysators verbunden wird. Die Zugabe der zusätzlich gefilterten Dialysierflüssigkeit in den Blutkreislauf wird als Dilution bezeichnet.

Zu Beginn der Dialysebehandlung müssen die in den Filterstufen angeordneten Filter mit Dialysierflüssigkeit durchspült und entlüftet werden.

Aus der DE 4027531 C1 ist eine Anordnung bekannt, die mittels eines Hydrophobfilter an einem oberen Ende eines Hohlfaserfilters eine Entlüftung erreicht. Bei dieser Anordnung ist nachteilig, dass sich das Hydrophobfilter über den gesamten Querschnitt des Hohlfaserfilters erstreckt und somit nicht Druckstabil ist. Des Weiteren ist eine derartige Membran nachteilig bezüglich der Herstellungskosten.

Aus der DE 3444671 A1 ist ein Verfahren bekannt, welches eine Entlüftung eines Filters mittels Sterilfilter und Hydrophobfilter erreicht. Der in zwei Kammern aufgeteilte zu entlüftende Filter hat dabei in der ersten Kammer an deren unteren Ende einen für die Zufuhr von Flüssigkeit vorgesehenen Anschluss, und am oberen Ende einen Anschluss mit einer Ausgleichskammer und dem besagten Hydrophobfilter. Die zweite Kammer hat am unteren Ende einen Anschluss, der mit dem besagten Sterilfilter verbunden ist und die für das Entlüften über dem besagten Hydrophobfilter angeordnet wird. Das Entlüften erfolgt nun derart, dass über den unteren Anschluss der ersten Kammer Flüssigkeit eingebracht wird und die in dieser Kammer befindliche Luft durch das Hydrophobfilter verdrängt. Sobald die gesamte Luft verdrängt ist, wird der Hydrophobfilter mit Flüssigkeit beaufschlagt und damit für weiteren Durchgang für Luft undurchlässig. Nun wird die Flüssigkeit durch die Membran des zu entlüftenden Filters gedrückt und beginnt die Luft in der zweiten Kammer durch das Sterilfilter zu verdrängen. Sobald im Wesentlichen keine Luft mehr durch das Sterilfilter entweicht, ist der Entlüftungsvorgang abgeschlossen. Nachteilig bei diesem Verfahren ist, dass das Sterilfilter nur einmalig verwendet werden kann.

Weiter nachteilig ist dabei, dass das Hydrophobfilter mit Flüssigkeit benetzt wird und somit die Sterilität und Druckfestigkeit fortan nicht mehr gewährleistet ist.

Wenn die Sterilität beeinträchtigt ist besteht die unmittelbare Gefahr, dass Verunreinigungen oder Keime wie Pilze und Bakterien von der Substitutionsleitung in das Blut des Patienten übergehen.

Auch nachteilig ist die mehrmalige notwendige manuelle Intervention von seitens des bedienenden Personals.

Aus WO 2006/049822 ist ein Verfahren zur Entlüftung eines Filters bekannt, bei dem der Filter aus der vertikalen Position heraus etwas geneigt wird. Damit wird erreicht, dass sich Luft am oberen Ende des Filters an der so entstandenen höchsten Stelle sammelt. Die Entlüftung kann nun über diese höchste Stelle erfolgen, wobei hier jedoch nachteilig eine manuell zu verschließende Klemme nach Entlüftung geschlossen werden muss.

Es ist bekannt, dass Hydrophobfilter Gase oder Gasgemische, insbesondere Luft und Dampf, durchlassen, nicht jedoch Flüssigkeiten und sonstige Feststoffe wie beispielsweise Bakterien und Toxine. Aufgrund dieser Eigenschaft werden Hydrophobfilter als Sterilfilter eingesetzt. Kommt die Membran des Hydrophobfilters mit Flüssigkeit in Berührung, so verliert sie die Eigenschaft der Gas- und Dampfdurchlässigkeit auch vollständig, so dass keinerlei Durchlässigkeit mehr gegeben ist. Darüber hinaus könne sich Bakterien an feuchte Stellen ansetzen und so an die feuchte Hydrophobmembran gelangen.

In einem Dialysierflüssigkeitskreislauf mit einem Hydrophobfilter und einem diesen abschließenden Anschluss muss der Anschluss nicht notwendig verschlossen werden, da das Hydrophobfilter druckbeständig ist und während der Behandlung normalerweise einen gegenüber dem Umgebungsdruck erhöhten Druck aufweist. Es ist jedoch vorteilhaft, das Hydrophobfilter nicht während der Behandlung alleinig als Druckbarriere zu benutzen. Dies ist insbesondere deshalb nachteilig, da während der Behandlung Druckschwankungen, hervorgerufen unter anderem durch Pumpen im Dialysierflüssigkeitskreislauf, eine wechselnde Druckbelastung an der Hydrophobfiltermembran hervorrufen und das Risiko des Brechens und damit verbunden ungewollten Flüssigkeitsaustritt und Probleme der Dilution am zu behandelnden Patient nach sich ziehen kann. Des Weiteren kann eine Hydrophobmembran so ihre Wirkung als Sterilfilter verlieren, so dass während der Behandlung ein erhöhtes Risiko für eine Kontamination des blutseitigen Kreislaufs besteht.

### Zusammenfassung

Der Erfindung liegt daher die Aufgabe zugrunde zumindest einen der genannten Nachteile zu überwinden sowie ein Entlüftungsventil und einen Filter mit einem zugehörigen Entlüftungsventil anzugeben, die eine automatische Entlüftung ermöglicht.

Diese Aufgabe wird mit dem Entlüftungsventil nach Anspruch 1 gelöst.

Vorteilhafte Weiterbildungen der Vorrichtung nach Anspruch 1 sind in den abhängigen Ansprüchen angegeben.

Offenbart wird ein Entlüftungsventil zum Entlüften eines Filters, vorzugsweise eines Filters für eine Blutbehandlungsmaschine etwa eines Filters für einen Dialysierflüssigkeitskreislauf einer Dialysemaschine. Das Entlüftungsventil umfasst einen Hohlraum mit einer ersten und einer zweiten Öffnung und einem Dichtelement im Innern des Hohlraums zum Abdichten der ersten Öffnung in einer ersten Position und zum Abdichten der zweiten Öffnung in einer zweiten Position. Das Dichtelement ist zwischen der ersten und der zweiten Position frei beweglich so dass ein Durchfluss zwischen der ersten und der zweiten Öffnung gebildet wird, wenn das Dichtelement sich in einer Zwischenposition zwischen der ersten und der zweiten Position befindet.

In einer Ausführungsform werden im Betrieb der Dialysemaschine und somit im Betrieb des Dialysierflüssigkeitskreislaufs eine senkrechte und eine horizontale Richtung sowie eine untere und eine obere Position vorgeben und das Dichtelement ist im Betrieb der Dialysemaschine und des Dialysierflüssigkeitskreislaufs so zwischen der oberen und der unteren Position beweglich, dass die Bewegungstrajektorie zwischen der unteren und der oberen Position eine Komponente in senkrechter Richtung aufweist. Mit anderen Worten: die erste Position ist eine untere Position und die zweite Position ist eine obere Position.

Weiterhin vorteilhaft ist das Dichtelement als Schwimmkörper zum Schwimmen auf der Dialysierflüssigkeit ausgebildet. Auf diese Weise trägt eine Auftriebskraft mit senkrechter, d.h. nach oben gerichteter Komponente zur Abdichtung des Dichtelements in der oberen Position bei.

In einer Ausführungsform umfasst die Oberflache des Dichtelements ein hydrophobes Material. Es kann aber auch das gesamte Dichtelement aus hydrophobem Material bestehen.

In der Anwendung im Zusammenhang mit einer Blutbehandlung, und/ oder einer Blutbehandlungsmaschine besteht ein Vorteil der offenbarten Vorrichtung darin, dass die Handhabung und die Fehlertoleranz der Bedienung verbessert und/ oder die notwendigen manuell auszuführenden Schritte des bedienenden Personals verringert. Weiterhin sind die Kosten für die Herstellung gemindert.

### Kurzbeschreibung der Zeichnungen

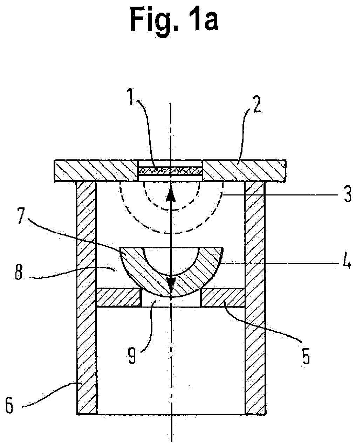

Figur 1a zeigt ein Entlüftungsventil im Einklang mit der Lehre der vorliegenden Erfindung.

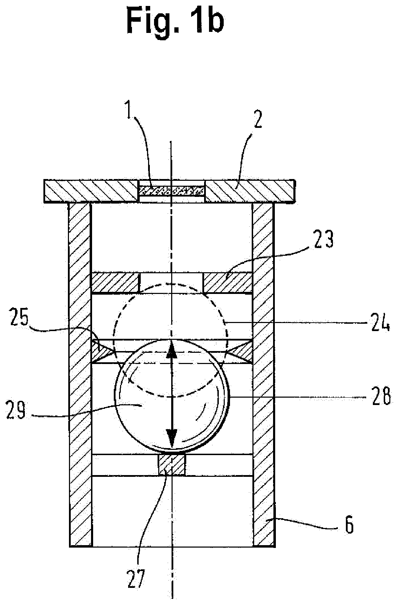

Figur 1b zeigt eine weitere Ausgestaltung eines Entlüftungsventils.

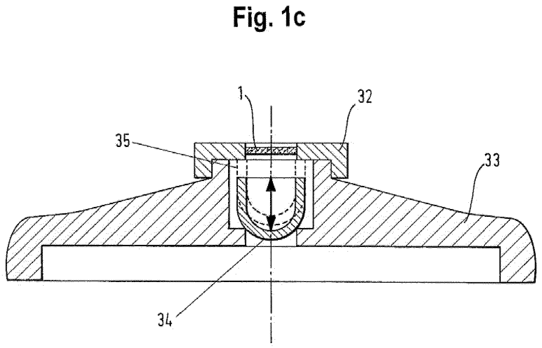

Figur 1c zeigt die Integration eines Entlüftungsventils in ein Gehäuse einer Filtervorrichtung.

### Detaillierte Beschreibung der Zeichnungen

Im Folgenden werden die Vorrichtung im Einklang mit der erfindungsgemäßen Lehre und vorteilhafte Weiterbildungen anhand der Abbildungen 1a, 1b und 1c näher erläutert, ohne dass die erfindungsgemäße Lehre darauf eingeschränkt sein soll.

Abbildung 1a zeigt ein Entlüftungsventil für einen Dialysierflüssigkeitskreislauf einer Dialysemaschine mit einer Hydrophobmembran 1, die vorteilhaft zugleich als Sterilfilter wirkt, mit einem Abschlusselement 2, das vorteilhaft als Aufnahmevorrichtung 2 für die Aufnahme der Hydrophobmembran 1 ausgebildet ist. Abschlusselement 2 ist mit dem bevorzugt innen zylindrisch geformten Körper 6, der nach beiden Richtungen hin offen ist und einen rohrförmigen Hohlraum bildet, verbunden und schließt diesen an einem ersten Ende vollständig ab. Am zweiten Ende ist der zylindrische Innenraum des Körpers 6 mittels des Abschlusselements 5 mit einer Öffnung 9, welches bevorzugt als ein scheibenförmiger Körper mit einem zentrisch axial verlaufenden angeordneten Loch ausgebildet ist, verbunden. Das Abschlusselement 5 schließt den Innenraum des Körpers 6 nach unten ab. In einer weiteren Ausgestaltung kann der Körper 6 auch bereits das Abschlusselement 5 mit ausformen. Im entstehenden Hohlraum 8 im inneren des Körpers 6 befindet sich ein beweglich angeordnetes Dichtelement 7, das so geformt ist, dass er durch Andrücken an das Abschlusselement 2 oder das Abschlusselement 5 einen gas-, dampf- und/oder flüssigkeitsdichten Verschluss bildet, wobei das Dichtelement 7 jeweils nur einen oder eines der beiden Abschlusselemente nicht jedoch beide zugleich verschließen kann. Im Betrieb der Dialysemaschine sind eine horizontale und eine vertikale Orientierung definiert, sowie eine obere Position 3 des Dichtelements 7 und eine untere Position 4 des Dichtelements 7. Im Betrieb der Dialysemaschine ist das Dichtelement 7 zwischen der unteren Position 4 und der oberen Position 3 beweglich. Die Bewegungstrajektorie zwischen der unteren Position 4 und der oberen Position 3 enthält somit zumindest eine Komponente in vertikaler Richtung. Um eine Bewegung des Dichtelements 7 entlang der Bewegungstrajektorie sicherzustellen können Führungsmittel (nicht gezeigt) vorgesehen sein. Das Dichtelement 7 ist vorzugsweise als Schwimmkörper zum Aufschwimmen auf einer Flüssigkeit ausgebildet, in der Anwendung in einem Dialysierflüssigkeitskreislauf: zum Aufschwimmen auf der Dialysierflüssigkeit.

Steigt in dem Körper 6 Flüssigkeit von unten nach oben an, so ist das Dichtelement 7 zunächst in Folge des Gewichts in der unteren Position 4 und an das Abschlusselement 5 vollständig verschließend aufliegend. Flüssigkeit und/oder das Gas und/oder der Dampf hebt anschließend das schwimmend ausgeführte Dichtelement 7 an und ermöglicht so, dass Gas durch die Membran des Hydrophobfilters 1 entweichen kann, bis das Dichtelement 7 in der Position 3 an das Abschlusselement 2 angedrückt wird und diese verschließt. Ein weiteres Austreten von Flüssigkeit, Dampf, und/oder Gas ist dann nicht mehr möglich. Bei der Ausführung des Dichtelements 7 als Schwimmkörper wirkt eine Auftriebskraft auf das Dichtelements 7, die der oberen Position 3 als Dichtkraft wirkt.

Vorteilhaft bei dieser Anordnung ist dabei, dass ein in einem angeschlossenen Fluidkreislauf durchgeführter Druckhaltetest keine Beschädigung der Hydrophobmembran verursacht.

Weiterhin vorteilhaft ist, dass eine kostengünstige Herstellung ermöglicht wird.

Weiterhin vorteilhaft im Zusammenhang mit der Anwendung des Entlüftungsventils zur Entlüftung eines Filters in einem Fluidkreislauf einer Blutbehandlungsmaschine, insbesondere in einer Dialysemaschine ist es, dass durch das Verschließen des Abschlusselements 5 in der unteren Position 4 kein Eindringen von Luft während des Betriebes der Blutbehandlung infolge von Druckschwankungen hervorgerufen werden kann und dass so keine Luft durch Pumpen zurück in den Filter gelangen kann.

Das Dichtelement 7 ist vorteilhaft aus elastischem Kunststoff ausgeführt, wobei die Oberfläche in vorteilhafter Weise aus einem hydrophoben Material besteht, oder mit diesem beschichtet ist. Damit wird erreicht, dass das Dichtelement 7 in Position 3 zusätzlich zur Auftriebskraft noch eine Kraft hervorgerufen durch die Oberflächenspannung erfährt und somit den Anpressdruck des Dichtelements 7 an die Aufnahmevorrichtung 2 und so die Dichtigkeit in Position 3 unterstützt.

Das Gewicht des Dichtelements 7 ist vorteilhaft so gewählt, dass bei Abwesenheit von Flüssigkeit in dem Körper 6 auf Grund der Gewichtskraft des Dichtelements 7 eine zum Abdichten in der unteren Position 4 ausreichende Dichtkraft vorhanden ist.

Abbildung 1b zeigt eine nicht zur Erfindung gehörende alternative Ausgestaltung zu der im Zusammenhang mit Figur 1a beschriebenen Vorrichtung. Gleiche oder entsprechende Elemente sind mit gleichen Bezugszeichen wie in Figur 1a versehen, wobei auf die Beschreibung der Figur 1a Bezug genommen wird an Stelle einer Widerholung. An die Stelle des Dichtelements 7 tritt ein kugelförmiges Dichtelement 29, das sich in einem bevorzugt zylindrischen Hohlraum des Körpers 6 im Wesentlichen auf und ab bewegen kann zwischen einer unteren Position 28, vorgegeben durch das Abschlusselement 27 und einer oberen Position 24, vorgegeben durch das Abschlusselement 2 oder in einer vorteilhaften Weiterbildung durch das zusätzlich angebrachte Begrenzungselement 23. Die Hydrophobmembran 1, das Abschlusselement 2, und der bevorzugt zylindrisch ausgeführte Körper 6 entsprechen den im Zusammenhang mit Figur 1a beschriebenen Elemente. Zusätzlich weist die

Anordnung von Figur 1b ein mit dem bevorzugt zylindrisch ausgeführten Körper 6 verbundenes Abschlusselement 27 auf. Die Abschlusselemente 2 und 23 können mit dem Körper 6 einstückig ausgeführt sein.

Ein Halteelement 25 ist zwischen dem Begrenzungselement 23 und dem Abschlusselement 27 derart angeordnet, dass, wenn sich das Dichtelement 29 in der Position 24 befindet, dieses die Öffnung in dem Begrenzungselement 23 und in dem Halteelement 25 vollständig gas-, dampf, und/oder flüssigkeitsfest verschließt und zusätzlich von dem Halteelement 25 in Position 24 gehalten wird, jedoch in der unteren Position 28 einen Durchfluss für Gase und Flüssigkeiten ausbildet. Das Halteelement 25 ist derart gestaltet, dass das Dichtelement 29 nur durch Beaufschlagung einer hinreichend großen Kraft von Position 24 nach Position 28 beziehungsweise von Position 28 nach Position 24 verschoben werden kann. Die Beaufschlagung der Kraft erfolgt vorteilhaft durch Druck, aufgebaut beispielsweise durch eine Pumpe in einem an das Entlüftungsventil abgeschlossenen Fluidkreislaufs. Bei der Entlüftung eines an das Entlüftungsventil angeschlossenen Filters wird zunächst Luft durch den Körper 6 von unten nach oben austreten und erst wenn Flüssigkeit das auf der Flüssigkeit aufschwimmende Dichtelement 9, soweit anhebt, dass es mit dem Dichtelement 25 gas- und flüssigkeitsdicht verschließt, kann über die Zufuhr weiterer Flüssigkeit der nötige Druck und damit die nötige Kraft aufgebaut werden, um das bewegliche Dichtelement 29 in Position 24 zu bewegen. Umgekehrt kann durch Erzeugung eines entsprechend hohen Unterdruckes das Dichtelement 29 von der Position 24 in die Position 28 bewegt werden und somit eine durchgängige Verbindung in dem Körper 6 hergestellt werden. Der bewegliche Dichtelement 29 und/oder das Haltelement 25 sind bevorzugt aus einem elastischen Material, beispielsweise weichem Kunststoff, Gummi oder Ähnlichem auszuführen. Der erforderliche Druck, um das Dichtelement 29 in die Position 24 zu bringen ist etwa durch die Wahl des Materials und der Abmessung des Dichtelements 29 und des Durchmessers der in dem Halteelement 25 vorgesehenen Öffnung vorteilhaft derart bemessen, dass er größer ist als die Druckschwankungen die während des Betriebs eines an das Entlüftungsventils angeschlossenen Fluidkreislaufs, etwa eines Dialysatkreislaufs einer Blutbehandlungsmaschine auftreten können. So kann ein versehentliches Öffnen verbunden mit ungewolltem Lufteintritt vermieden werden. Andererseits sollte der erforderliche Druck geringer als der maximal durch die angeschlossene Vorrichtung erzeugbare Druck sein. Damit kann auf vorteilhafte Weise erreicht werden, dass die Vorrichtung automatisch durch das Gerät ohne zusätzliche Vorrichtungen an der Gerätevorderseite bedient werden kann, wenn das Entlüftungsventil auf der Geräterückseite angeordnet ist. Weiters vorteilhaft ist, dass das Ventil wieder geöffnet werden kann und damit bei der Anwendung in einem Dialysatkreislauf einer Dialysemaschine nach der Behandlung und der Re-Infusion ein rascheres und rückstandfreieres Entleeren erfolgt.

Abbildung 1c zeigt in einer vorteilhaften Ausgestaltung eine vorteilhafte Integration der Vorrichtung entsprechend der Abbildung 1a, in einer Variante, in das Gehäuse der Filtervorrichtung, bevorzugt in einen Deckel eines Filtergehäuses.

In ähnlicher Weise wie in den zuvor beschriebenen Vorrichtungen, weist diese Vorrichtung der Figur 1c eine Hydrophobmembran 1 die zugleich ein Sterilfilter sein kann, ein als Vorrichtung für die Aufnahme der Hydrophobmembran 1 ausgebildetes Abschlusselement 32, welches mit dem bevorzugt innen zylindrisch geformten Grundkörper 33, der zugleich auch bevorzugt den Deckel eines Filtergehäuses darstellt, der nach beiden Richtungen hin offen ist, verbunden ist und diesen an einem ersten Ende, bevorzugt dem oberen Ende, vollständig abschließt. Das bewegliche Dichtelement in der Abbildung 1c in Position 34 eingezeichnet, kann sich jedoch entlang des bevorzugt zylindrischen Hohlraumes in dem Grundkörper 33 im Wesentlichen entlang einer bevorzugt senkrecht verlaufenden Achse von Position 34 bis Position 35 bewegen. Die beiden Positionen sind dabei derart vorgegeben, dass in Position 35 der bewegliche Teil unter Anpressung an das Abschlusselement 32 einen luft-, dampf- und/oder flüssigkeitsdichten Verschluss bildet und in Position 34 das bewegliche Dichtelement einen luft-, dampf- und/oder flüssigkeitsdichten Verschluss unter Anpressung an das Abschlusselement 32 bildet. Die Kraft mit der der bewegliche Dichtelement 32 angepresst wird, ergibt sich in Position 35 aus der Druckdifferenz zwischen Innen- und Aussenraum, sowie der Auftriebskraft des aufschwimmenden Dichtelements abzüglich der Gewichtskraft, in Position 34 ergibt sich die Anpresskraft aus der Gewichtskraft und der Druckdifferenz.

## Patentansprüche

1. Filter für einen Dialysierflüssigkeitskreislauf mit einer Hydrophobmembran, **gekennzeichnet durch** ein Entlüftungsventil zum Entlüften des Filters, umfassend einen Hohlraum (8) mit einer ersten (9) und einer zweiten Öffnung (1), und einem Dichtelement (7, 29) im Innern des Hohlraums zum Abdichten der ersten Öffnung in einer ersten Position (4, 28) und zum Abdichten der zweiten Öffnung in einer zweiten Position (3, 24), wobei das Dichtelement zwischen der ersten (4, 28) und der zweiten (4, 28) Position frei beweglich ist und wobei ein Durchfluss zwischen der ersten und der zweiten Öffnung (1) gebildet wird, wenn das Dichtelement (7, 29) sich in einer Zwischenposition zwischen der ersten (4, 28) und der zweiten (3, 24) Position befindet, wobei das Dichtelement (7, 29) derart ausgebildet ist, um in einer Flüssigkeit an der Oberfläche zu schwimmen, wobei die zweite Öffnung (1) mit der Hydrophobmembran versehen ist.

2. Filter nach Anspruch 1, wobei im Betrieb des Dialysierflüssigkeitskreislaufs eine senkrechte und eine horizontale Richtung vorgegeben werden und wobei das Dichtelement (7, 29) im Betrieb des Dialysierflüssigkeitskreislaufs mit einer Bewegungskomponente in senkrechter Richtung zwischen der ersten (4, 28) zur zweiten (3, 24) Position beweglich ist.

3. Filter nach Anspruch 1 oder 2, wobei eine Oberfläche des Dichtelements (7, 29) ein hydrophobes Material umfasst.

4. Filter nach einem der vorangehenden Ansprüche, wobei das Dichtelement (7) eine Becherform oder die Form einer Halbkugelschale oder die Form einer Kugel aufweist.

5. Filter nach Anspruch 4, wobei das Dichtelement eine Führungslippe zum Führen des Dichtelements zwischen der ersten und zweiten Position aufweist.

6. Filter nach einem der vorangegangen Ansprüche, wobei das Dichtelement (29) an der zweiten Position (24) durch eine durch Druck lösbare Einrastung verschließend festgehalten wird.

7. Filter nach Anspruch 6, wobei das Material des Dichtelements (29) elastische Eigenschaften aufweist.

8. Filter nach einem der vorangehenden Ansprüche, wobei das Entlüftungsventil in einer Entlüftungskappe (33) des Filters integriert ist.

9. Dialysierflüssigkeitskreislauf zur Zubereitung von Dialysierflüssigkeit mit einem Filter nach einem der vorangehenden Ansprüche.

## Claims

1. A filter for a dialysis fluid circulation, having a hydrophobic membrane, **characterized by** an air-relief valve for venting the filter, comprising a cavity (8) with a first opening (9) and a second opening (1), and a sealing element (7, 29) in the interior of the cavity for sealing the first opening in a first position (4, 28) and for sealing the second opening in a second position (3, 24), wherein the sealing element is freely movable between the first position (4, 28) and the second position (3, 24), and wherein a flow is formed through the first opening and the second opening (1), when the sealing element (7, 29) is in an intermediate position between the first position (4, 28) and the second position (3, 24), wherein the sealing element (7, 29) is designed to float at the surface of a liquid, wherein the second opening (1) is provided with the hydrophobic membrane.

2. The filter according to claim 1, wherein during operation of the dialysis fluid circulation, a vertical direction and a horizontal direction predetermined, and wherein the sealing element (7, 29) is movable during operation of the dialysis fluid circulation, with one component of movement being in a vertical direction between the first position (4, 28) and the second position (3, 24).

3. The filter according to claim 1 or 2, wherein a surface of the sealing element (7, 29) comprises a hydrophobic material.

4. The filter according to any one of the preceding claims, wherein the document (7) has a cup shape or is in the form of a hemispherical shell or in the form of a sphere.

5. The filter according to claim 4, wherein the sealing element has a guide lip for guiding the sealing element between the first and second positions.

6. The filter according to any one of the preceding claims, wherein the sealing element (29) is secured and locked in the second position (24) by a pressure-releasable lock.

7. The filter according to claim 6, wherein the material of the sealing element (29) has elastic properties.

8. The filter according to any one of the preceding claims, wherein the air-relief valve is integrated into a ventilation cap (33) on the filter.

9. A dialysis fluid circulation for preparation of dialysis fluid with a filter according to any one of the preceding claims.

## Revendications

1. Filtre pour circuit de liquide de dialyse, comportant une membrane hydrophobe, **caractérisé par** une soupape de purge d'air pour la purge du filtre, comprenant une cavité (8) dotée d'une première (9) et d'une seconde (1) ouverture, et un élément d'étanchéité (7,29) à l'intérieur de la cavité pour colmater la première ouverture dans une première position (4,28) et pour colmater la seconde ouverture dans une seconde position (3,24), l'élément d'étanchéité étant librement mobile entre la première (4,28) et la seconde (4,28) position et un écoulement traversant étant établi de la première et la seconde ouverture (1) lorsque l'élément d'étanchéité (7,29) se trouve dans une position intermédiaire entre la première (4,28) et la seconde (3,24) position, l'élément d'étanchéité (7,29) étant réalisé de manière à flotter dans un liquide à la surface, la seconde ouverture (1) étant pourvue de la membrane hydrophobe.

2. Filtre selon la revendication 1, dans lequel, en cours de fonctionnement du circuit de liquide de dialyse, un sens vertical et un sens horizontal sont prédéfinis et l'élément d'étanchéité (7,29), en cours de fonctionnement du circuit de liquide de dialyse, est mobile grâce à un composant de mobilité dans le sens vertical entre la première (4,28) et la seconde (3,24) position.

3. Filtre selon la revendication 1 ou 2, dans lequel une surface de l'élément d'étanchéité (7,29) comprend un matériau hydrophobe.

4. Filtre selon une des revendications précédentes, dans lequel l'élément d'étanchéité (7) présente une forme de godet ou la forme d'une coque en demi-sphère ou la forme d'une sphère.

5. Filtre selon la revendication 4, dans lequel l'élément d'étanchéité présente une lèvre de guidage pour guider l'élément d'étanchéité entre la première et la seconde position.

6. Filtre selon une des revendications précédentes, dans lequel l'élément d'étanchéité (29) est maintenu à la seconde position (24) en obturation par un enclenchement dissociable par pression.

7. Filtre selon la revendication 6, dans lequel le matériau de l'élément d'étanchéité (29) présente des propriétés élastiques.

8. Filtre selon une des revendications précédentes, dans lequel la soupape de purge est intégrée dans un capuchon de purge (33) du filtre.

9. Circuit de liquide de dialyse pour la préparation de liquide de dialyse, comportant un filtre selon une des revendications précédentes.
